# EUROPEAN PATENT APPLICATION

(11) **EP 3 957 370 A1**
(43) Date of publication of application: **23.02.2022**
(21) Application number: 20217687.1
(22) Date of filing: 29.12.2020
(51) Int. Cl.: A62B 18/02, A61L 9/20, A62B 23/02, A41D 13/11

(54) **AIR PURIFICATION MASK**

(30) Priority: 19.08.2020 HK 32020014515
(71) Applicant: John Technology Holdings Limited, Kwun Tong, Kowloon (HK)
(72) Inventor: YUEN, Se Kit, Kowloon (HK)
(74) Representative: Murgitroyd & Company

(57) **Abstract**

The present invention provides a mask. The mask includes a mask body, a filter module installed on the mask body, a light source and a photocatalyst plate. The filter module includes a cavity, and it is provided with an air inlet and an air outlet which are located in different regions of the filter module. The photocatalyst plate and the light source are both located in the cavity. The surface of the photocatalyst plate is covered with a photocatalyst coating, and the light emitted by the light source irradiates the photocatalyst plate. In the embodiment of the present invention, by placing the light source and the photocatalyst plate in the cavity of the filter module, the photocatalyst generates strong oxidizing substances under light irradiation, which substances can inactivate harmful substances. Therefore, by placing the light source and the photocatalyst plate in the cavity of the filter module, harmful substances entering the mask can be sterilized and inactivated. The harmful substances after the sterilization and inactivation are no longer harmful, so there is no secondary pollution caused by harmful substances dropping off. It solves the problem of secondary pollution caused by the harmful substances in existing masks.

## Description

### FIELD OF THE DISCLOSURE

The invention relates to the field of air purification, in particular to a mask.

### BACKGROUND

In the prior art, masks mainly absorb particulate dust and toxic or harmful substances in the air through an activated carbon filter or a meltblown fabric cloth, so as to prevent the particulate dust and toxic or harmful substances in the air from being breathed in. However, the toxic or harmful substances adsorbed by the activated carbon filter layer or meltblown fabric cloth can easily be separated from the mask to form secondary pollution. That is, the mask in the prior art only adsorbs harmful substances, which are easily dropped off from the masks to cause secondary pollution.

### SUMMARY

The present invention is achieved to solve the above-described problem, an embodiment of the present invention provides a mask comprising: a mask body and a filter module attached on the mask body. The filter module includes a cavity, an air inlet and an air outlet arranged on the cavity, and a light source and a photocatalyst plate installed in the cavity ; The surface of the photocatalyst plate is coated with a photocatalyst layer, and the light emitted by the light source is irradiated on the photocatalyst.

Optionally, the filter module further includes an air filter installed at the air inlet. Next, the filter module further includes a reflector, which reflector is located in the cavity, and the reflector reflects the light emitted by the light source. The light source is a short-wave ultraviolet generator.

Next, the mask further includes a protective cover, which protective cover is located outside the filter module and cover the air filter.

Next, the filter module further includes a power interface provided outside the filter module and a rechargeable battery provided in the cavity. The rechargeable battery and the power interface is electrically connected to the light source.

Next, the filter module further includes a control device and indicator lights located in the cavity, and the filter module is provided with openings adapted to the indicator lights to enable the indicator lights to be exposed on the surface of the filter module. The control device is electrically connected to the light source.

Next, a gasket is further provided around and connected to the mask body.

Next, the mask body is further provided with a first magnet and the filter module is provided with a metal sheet, such that the filter module and the mask body are attached through magnetic attraction of the magnet and the metal sheet.

Next, a second magnet is provided on the side of the filter module corresponding to the protective cover.

In the embodiment of the present invention, by placing the light source and the photocatalyst plate with its surface coated with photocatalyst in the cavity of the filter module, light emitted from the light source will irradiate the photocatalyst plate. Since the photocatalyst produces strong oxidizing substances under light irradiation, it can effectively degrade toxic or harmful substances in the air, such as various bacteria and viruses, and can decompose or render harmless the toxic substances released by bacteria or viruses, making it no longer harmful to the human body. Therefore, by placing the light source and the photocatalyst plate in the cavity of the filter module, the toxic or harmful substances on the mask can be sterilized and inactivated. The toxic or harmful substances after the sterilization and inactivation are no longer toxic, and therefore their dropping off will not cause secondary pollution. It solves the problem that the mask in the prior art only absorbs particulate dust and toxic or harmful substances, which substances are easily dropping off to cause secondary pollution.

### BRIEF DESCRIPTION OF THE DRAWINGS

In order to more clearly describe the technical solutions in the embodiments of the present invention, the following are descriptions of the accompanying drawings. Obviously, the following drawings are only embodiments of the present invention. For those of ordinary skill in the art, without any creative work they may produce other drawings based on the following drawings.
FIG.1 is a front view of a mask provided by an embodiment of the present invention;
FIG.2 is a left side view of a mask provided by an embodiment of the present invention;
FIG.3 is a right side view of a mask provided by an embodiment of the present invention;
FIG.4 is a top view of a mask provided by an embodiment of the present invention;
FIG.5 is a bottom view of a mask provided by an embodiment of the present invention;
FIG.6 is a rear view of a mask provided by an embodiment of the present invention;
FIG.7 is a first perspective view of the appearance of a mask provided by an embodiment of the present invention;
FIG.8 is a second perspective view of the appearance of a mask provided by an embodiment of the present invention;
FIG.9 is the first cross-sectional view of the mask provided by the embodiment of the present invention;
FIG.10 is the second cross-sectional view of the mask provided by the embodiment of the present invention;
FIG.11 is the third cross-sectional view of the mask provided by the embodiment of the present invention;
FIG.12 is an exploded perspective view of a mask provided by an embodiment of the present invention.

### DESCRIPTION OF AN EMBODIMENT

The technical solutions in the embodiments of the present invention will be clearly and completely described below in conjunction with the accompanying drawings. Obviously, the described embodiments are only a part of the embodiments of the present invention, not all the embodiments. On the basis of the embodiments of the present invention, all other embodiments obtained by those of ordinary skill in the art without creative work shall fall within the protection scope of the present invention.

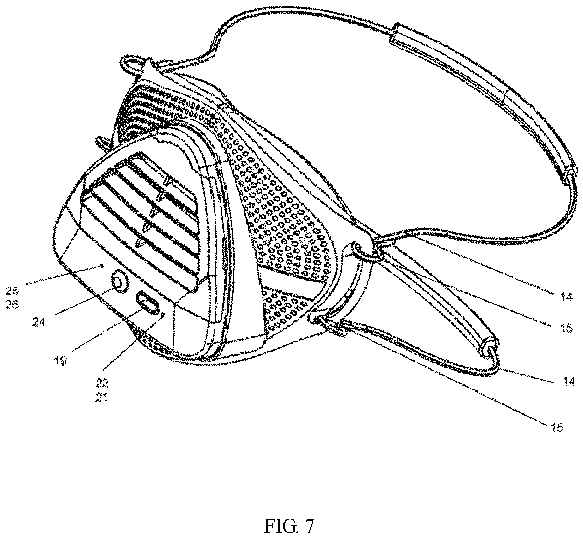

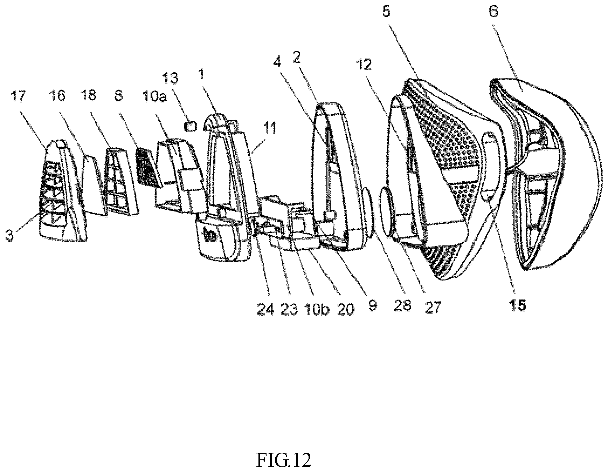

Referring to FIG.7 and FIG.12, the mask provided by the embodiment of the present invention includes a mask body 5 and a filter module 29 attached to the mask body 5; The filter module 29 includes a cavity 7, an air inlet 3 and an air outlet 4 arranged on the cavity 7, and a light source 9 and a photocatalyst plate 8 also arranged in the cavity 7; The surface of the photocatalyst plate 8 is coated with a photocatalyst layer. The light emitted by the light source 9 irradiates the photocatalyst plate 8.

Specifically, the material of the mask body 5 provided by the embodiment of the present invention may be a plastic material with a certain strength. When the user wears the mask, the user can apply a slight external force to the mask body 5 to cause plastic deformation of the mask body 5 to make the mask body 5 fit tighter to the shape of the user's face to form a closed breathing space. The shape of the mask body 5 may be an ellipse or the like, which is set to cover the average shape of users for close fitting. The filter module 29 can be composed of the first half-shell 1 and the second half-shell 2 both made of plastic material and fixedly connected to each other by fasteners such as screws. There is a cavity 7 between the two half shells. The first half shell 1 is provided with an air inlet 3, and the second half shell 2 is provided with an air outlet 4. An air intake grille 12 is provided on the mask body 5 at a position corresponding to the air outlet 4.

The shape of the photocatalyst plate 8 can be set according to actual needs, for example, the shape can be quadrilateral. The photocatalyst plate 8 can be made of plastic or metal. The surface of the photocatalyst plate 8 can be uniformly covered with a nano-photocatalyst layer by coating. A bracket 11 is provided at the air inlet 3 in the cavity. The bracket 11 can be fixed and welded to the inner side of the first half shell 1, and the photocatalyst plate 8 is fixed at the bracket 11. The cavity 7 may be provided with an embedded slot or an embedded column, such that the photocatalyst plate 8 can be firmly fixed.

The type of light emitted by the light source 9 can be any type of light that can cause the photocatalyst plate 8 to degrade toxic or harmful substances. The light source 9 is electrically connected to a power source for supply of power. The power source may be a disposable power source or a rechargeable power source built into the air purifier. The light source 9 is arranged next to the photocatalyst plate 8 and is located between the photocatalyst plate 8 and the air outlet 4. The light source 9 is positioned such that the light emitted can fully irradiate the photocatalyst plate 8. The cavity 7 may be provided with an embedded slot or an embedded post for fixing the light source 9.

When the user uses the mask provided by the embodiment of the present invention to inhale, external air enters through the air inlet 3, flows through the photocatalyst plate 8 in the cavity, flows out from the air outlet 4, and enter the breathing space formed by the mask body 5 and the user's face for breathing by the user. The photocatalyst produces strong oxidizing substances under the irradiation of light, which effectively degrades toxic or harmful substances in the air, such as various bacteria and viruses, and decomposes and renders harmless the toxic substances released by the bacteria or viruses, so that they are not harmful to the human body. Therefore, by placing the light source 9 and the photocatalyst plate 8 in the cavity 7 of the filter module 29, the toxic or harmful substances on the mask can be sterilized and inactivated, such that the substances are no longer toxic and will not cause any secondary pollution. It solves the problem that the mask in the prior art only absorbs particulate dust and toxic or harmful substances that might easily dropped off to cause secondary pollution.

In addition, the mask in the prior art does not have the function of disinfection and sterilization. So when exhaled air is discharged into the air with germs from the user, it may result in cross infection. However when the user breathes out using the mask provided by the embodiment of the present invention, the exhaled air enters from the intake grill 12, flows through the cavity 7 with the photocatalyst plate 8 and flows out from the air inlet 3. Since the exhaled air flows through the photocatalyst plate 8 irradiated by light emitted from the light source 9, the photocatalysis can turn the toxic or harmful substances harmless. The exhaled air with toxic or harmful substances, such as bacteria or viruses, are sterilized and inactivated in the cavity 7 before being discharged into the surrounding. It solves the problem that the mask in the prior art does not have the function of disinfecting or sterilizing the exhaled air with germs which cause cross infection.

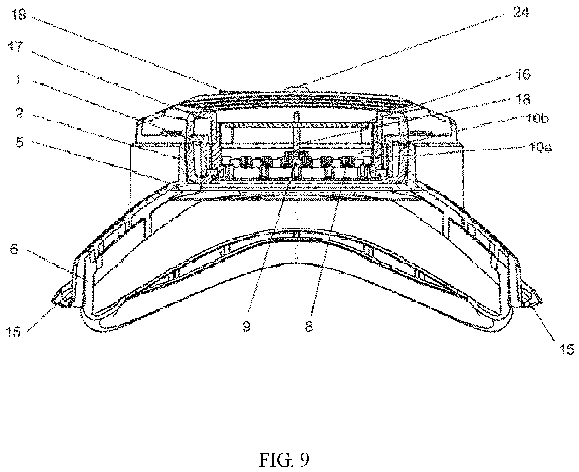

Optionally, as shown in FIG.9 and FIG.12, the filter module 29 further includes an air filter 16 arranged at the air inlet 3. Specifically, the air filter 16 and the filter module 29 can be detachably connected. The air filter 16 can be placed on a filter holding frame 18 installed on the outside of the first half shell 1. The air filter 16 can be made of polypropylene melt-blown non-woven fabric, the fiber diameter of which melt-blown non-woven fabric about 2 µm is relatively small and may effectively block bacteria and other toxic or harmful substances from the outside of the mask. By setting the air filter 16 to filter out toxic or harmful substances such as dust, bacteria and viruses in the air, the air filtering effect of the mask can be further improved.

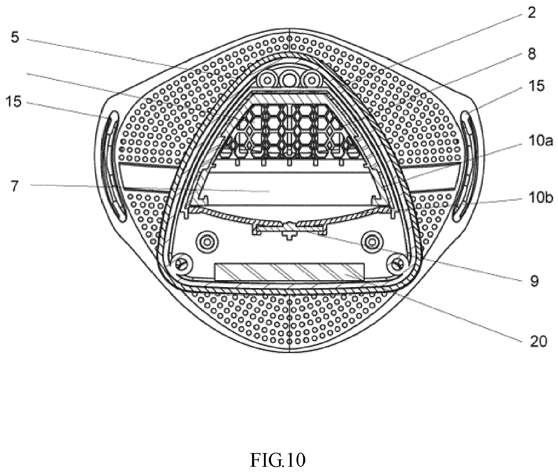

Next, as shown in FIG.10 and FIG.12, the filter module 29 further includes a reflector, the reflector is located in the cavity 7. The reflector reflects the light emitted by the light source 9.

Specifically, the reflector may include a first reflecting component 10a and a second reflecting component 10b. The first reflecting component 10a is installed at the bracket 11 around the air inlet 3 surrounding the photocatalyst plate 8. The first reflecting component 10a may be assembled by four flat mirrors namely the first mirror, the second mirror, the third mirror, and the fourth mirror connected in series. The reflecting surfaces of these four mirrors are all facing the photocatalyst plate 8. The second reflecting component 10b may be a plane mirror, which is located in the same side of the light source 9 and facing directly opposite to the reflective surfaces of the first reflecting component 10a.

By setting the reflector to reflect the light emitted by the light source 9, such as short-wave ultraviolet light, the light intensity can be enhanced, thereby enhancing the efficiency of the photocatalyst plate 8 to degrade toxic or harmful substances, and thereby enhancing the sterilization effect of the mask.

Next, the light source 9 is a short wave ultraviolet generator. Specifically, a light source 9 that generates short-wave ultraviolet light can be selected, such as UVC lamp beads (UVC is short-wave ultraviolet light). The short-wave ultraviolet light irradiates the photocatalyst coating which will generate strong oxidizing substances to effectively degrade poisonous or harmful substances like bacteria and viruses in the air. In addition, short-wave ultraviolet rays can also destroy the molecular structure of DNA (deoxyribonucleic acid) or RNA (ribonucleic acid) in microbial cells, causing death of their growth cells and/or regenerative cells. Choosing the short-wave ultraviolet light for the light source 9 can therefore further enhance the sterilization and disinfection effect of the mask because the short-wave ultraviolet light by itself has a sterilizing and disinfecting function.

As shown in FIG.12, the mask further includes a protective cover 17 which is located outside the filter module 29 and also outside the air filter 16. Specifically, the protective cover 17 can be detachably connected to the filter module 29. The protective cover 17 may be made of plastic material, and a grille is provided on the protective cover 17 at a position corresponding to the air inlet 3. The protective cover 17 can prevent foreign objects from impacting and damaging the internal components of the filter module 29. In addition, the protective cover 17 is detachably connected to the filter module 29, which is convenient for the user to remove the protective cover 17 to clean or replace internal components such as the air filter 16 and the reflector in the filter module 29, thereby increasing the service life of the mask.

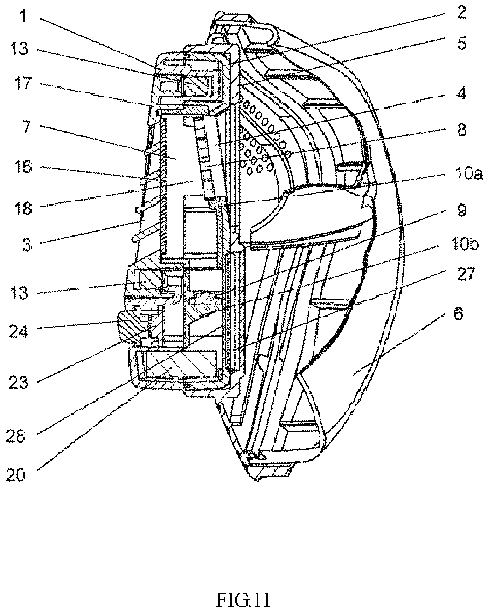

Next, as shown in FIG.11 and FIG.12, the filter module 29 further includes a power interface 19 arranged outside the filter module 29 and a rechargeable battery 20 arranged in the cavity 7. The rechargeable battery 20 is electrically connected to the power interface 19 and the light source 9 respectively. The user can charge the rechargeable battery 20 through the power interface 19, and the rechargeable battery 20 provides power to the light source 9. The rechargeable battery 20 is used to supply power to the light source 9, avoiding the use of disposable batteries which might cause pollution to the environment when discarded.

Next, as shown in FIG.12, the filter module 29 further includes a control device and indicator lights located in the cavity 7. The filter module 29 is provided with openings adapted to the indicator lights which are exposed on the surface of the filter module 29. The control device is electrically connected to the light source. 9.

Specifically, the control device includes a power supply circuit board 23 and a switch 24. The power supply circuit board 23 is electrically connected to the rechargeable battery 20. The switch 24 may be provided on the power supply circuit board 23 and electrically connected to the power supply circuit board 23 for controlling the on and off of the light source 9. The switch 24 may be a mechanical switch such as a button switch or a rotary switch, etc. The first half shell 1 is provided with an opening corresponding to the switch 24, so that the switch 24 is exposed on the surface of the filter module 29, to make it convenient for the user to control.

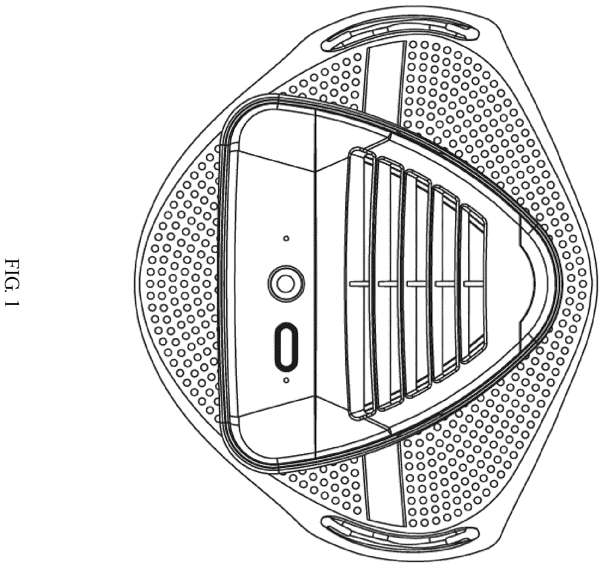

Further, as shown in FIG.1 and FIG.7, the filter module 29 further includes a first indicator light 21 and a second indicator light 26. The filter module 29 is provided with a first light hole 22 and a second light hole 25 respectively corresponding to the first indicator light 21 and the second indicator light 26, so that the light information of the indicator lights can be sent to the outside through the light hole.

Specifically, the first indicator light 21 and the second indicator light 26 may be LED lights (LEDs are light-emitting diodes), and the LED lights may be soldered on the power supply circuit board 23 and electrically connected to the power supply circuit board 23. The status of the light source 9 or the rechargeable battery 20 is indicated by blinking, brightness, or colors of LEDs. For example, the first indicator light 21 can indicate any of the following states by blinking, being bright or dark, or showing different colors:
The rechargeable battery 20 is being charged;
The rechargeable battery 20 has low power;
The rechargeable battery 20 is fully charged.

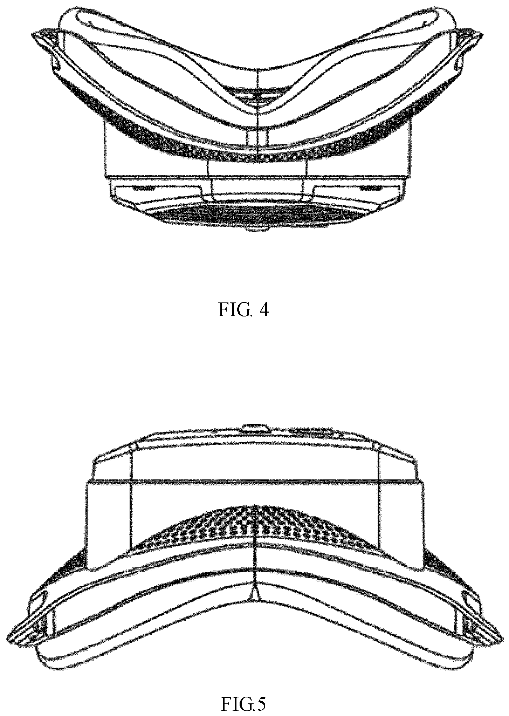

As shown in FIG.4 and FIG.5, a gasket 6 is further provided around the mask body 5, and the gasket 6 is connected to the mask body 5. Specifically, the gasket 6 can be made of plastic or medical sponge. The gasket 6 may be provided with a groove, through which an elastic connection between the gasket 6 and the periphery of the mask body 5 can be realized. The gasket 6 can make the mask body 5 fit to the user's face closely, forming a more tightly closed breathing space, and preventing toxic or harmful substances in the air from entering the breathing space through the gap between the mask body 5 and the user's face.

Next, the mask body 5 is further provided with a first magnet 27, and the filter module 29 is provided with a metal sheet 28. The filter module 29 and the mask body 5 are connected through magnetic attraction of the metal sheet 28 and the first magnet 27. The detachable connection between the mask body 5 and the filter module 29 is realized by the magnetic attraction of the metal sheet 28 and the first magnet 27, so that the user can disassemble the filter module 29 and the mask body 5 for cleaning separately.

Next, a second magnet 13 is provided on the side of the filter module 29 opposite to the protective cover 17. Specifically, the second magnet 13 is fixedly connected to the filter module 29, a metal plate may be provided on the protective cover 17 at a position opposite to the second magnet 13, and the filter module 29 and the protective cover 17 are connected by magnetic attraction between the second magnet 13 and the metal plate, such that detachable connection is achieved.

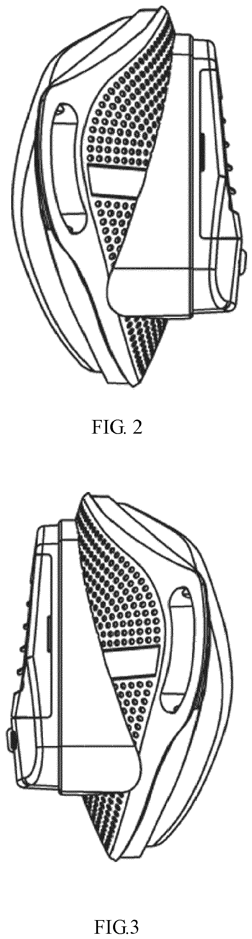

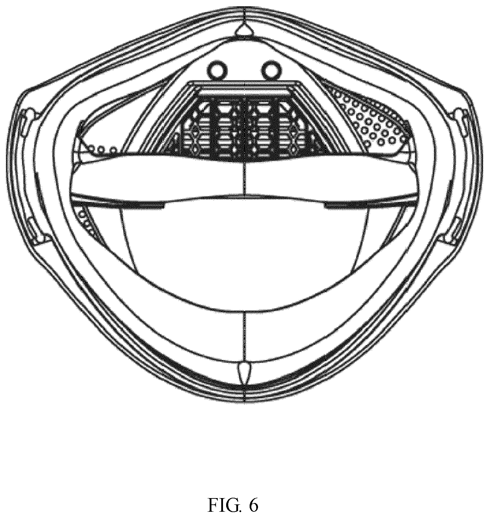

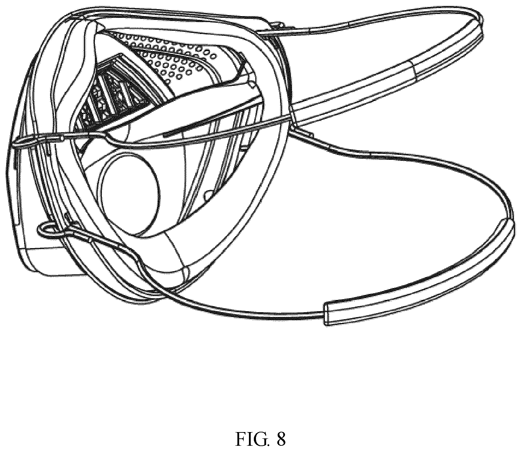

Furthermore, as shown in FIG.2, FIG.3, FIG.6 and FIG.8, the opposite sides of the mask body 5 are also provided with belt holes 15, and the elastic band 14 with an adjustable length passes through the belt holes 15. When the user wears the mask, by adjusting the length of the elastic band 14 the user can fit the mask to face closely to prevent the possibility of toxic or harmful substances entering the breathing space through the gap between the mask body 5 and the user's face. It also reduces the possibility of users spreading bacteria and viruses through breathing out air with germs.

Further, the mask body 5 may be provided with uniformly arranged circular recessed holes, which can increase the friction coefficient of the surface of the mask body 5, thereby producing a non-slip effect when worn by the user.

The embodiments of the present invention are described above with reference to the accompanying drawings, but the present invention is not limited to the above-mentioned specific embodiments. The above-mentioned specific embodiments are merely illustrative and not restrictive. Under the enlightenment of the present invention, those of ordinary skill in the art can make many forms of changes without departing from the purpose of the present invention and the protection scope of the claims, all of which fall within the protection of the present invention.

## Claims

1. A mask, **characterized in that** the mask comprises a mask body and a filter module provided on the mask body. The filter module includes a cavity, an air inlet and an air outlet arranged on the cavity, and a light source and a photocatalyst plate installed in the cavity. The surface of the photocatalyst plate is coated with a photocatalyst layer, and the light emitted by the light source irradiates the photocatalyst module.

2. The mask according to claim 1, wherein the filter module further comprises an air filter installed at the air inlet.

3. The mask according to claim 1, wherein the filter module further comprises a reflector, which reflector is located in the cavity, and the reflector reflects the light emitted by the light source.

4. The mask according to claim 1, wherein the light source is a short-wave ultraviolet generator.

5. The mask according to claim 2, wherein the mask further comprises a protective cover, which protective cover is located outside the filter module and cover the air filter.

6. The mask according to claim 1, wherein the filter module further comprises a power interface provided outside the filter module and a rechargeable battery provided in the cavity. The rechargeable battery and the power interface is electrically connected to the light source.

7. The mask according to claim 1, wherein the filter module further comprises a control device and indicator lights located in the cavity, and the filter module is provided with openings adapted to the indicator lights to enable the indicator lights to be exposed on the surface of the filter module. The control device is electrically connected to the light source.

8. The mask according to claim 1, wherein a gasket is provided around the mask body, and the gasket is connected to the mask body.

9. The mask according to claim 1, wherein the mask body is further provided with a first magnet and the filter module is provided with a metal sheet, such that the filter module and the mask body are attached through magnetic attraction of the magnet and the metal sheet.

10. The mask according to claim 5, wherein a second magnet is provided on the side of the filter module corresponding to the protective cover.
